(19) Europäisches Patentamt
European Patent Office
Office européen des brevets

(11) Veröffentlichungsnummer: 0 370 499
A2

(12) # EUROPÄISCHE PATENTANMELDUNG

(21) Anmeldenummer: 89121593.1

(51) Int. Cl.5: A61K 31/47

(22) Anmeldetag: 23.11.89

(30) Priorität: 24.11.88 DE 3839659

(43) Veröffentlichungstag der Anmeldung:
30.05.90 Patentblatt 90/22

(84) Benannte Vertragsstaaten:
AT BE CH DE ES FR GB GR IT LI LU NL SE

(71) Anmelder: BOEHRINGER INGELHEIM KG

D-6507 Ingelheim am Rhein(DE)
(84) BE CH DE ES FR GR IT LI LU NL SE AT

Anmelder: BOEHRINGER INGELHEIM INTERNATIONAL G.M.B.H.

D-6507 Ingelheim am Rhein(DE)
(84) GB

(72) Erfinder: Carter, Adrian, Dr.
Pfarrer-Heberer-Strasse 34a
D-6530 Bingen(DE)
Erfinder: Ensinger, Helmut, Dr. Dipl.-Chem.
Schützenpfad 16
D-6507 Ingelheim am Rhein(DE)
Erfinder: Merz, Herbert, Dr. Dipl.-Chem.
Rotweinstrasse 53
D-6507 Ingelheim am Rhein(DE)
Erfinder: Müller, Enzio, Prof.
Grundstrasse 22
D-6507 Ingelheim am Rhein(DE)
Erfinder: Stransky, Werner, Dr. Dipl.-Chem.
Im Hippel 24
D-6535 Gau-Algesheim(DE)
Erfinder: Walther, Gerhard, Dr. Dipl.-Chem.
Pfarrer-Heberer-Strasse 37
D-6530 Bingen(DE)

(54) **Verwendung von Benzomorphanen zur Zytoprotektion.**

(57) Die Erfindung betrifft die Verwendung von Benzomorphanderivaten und ihren pharmakologisch unbedenklichen Säureadditionssalzen zur Zytoprotektion.

EP 0 370 499 A2

## Verwendung von Benzomorphanen zur Zytoprotektion

Die Erfindung betrifft die Verwendung von Benzomorphanen der allgemeinen Formel I

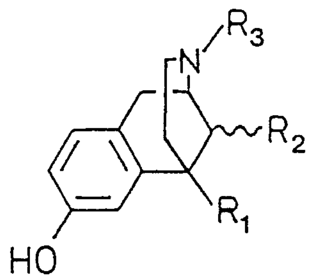

I

worin

$R_1$ Methyl, Ethyl;

$R_2$ Methyl, Ethyl;

$R_3$ 2-Methoxyethyl, 2-Methoxypropyl, 2-Furylmethyl (Furfuryl), 2-Tetrahydrofurylmethyl (Tetrahydrofurfuryl),

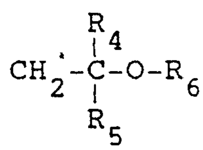

;

$R_4$ Wasserstoff, Methyl;

$R_5$ Wasserstoff, Methyl;

$R_6$ Methyl, Ethyl, Propyl, Isopropyl, Phenyl

bedeuten können;

deren α und β-Formen sowie deren enantiomere Formen und den entsprechenden pharmakologisch unbedenklichen Säureadditionssalzen zur Zytoprotektion.

Die Benzomorphanderivate der allgemeinen Formel I weisen mindestens 3 C-Atome mit einem Asymmetriezentrum auf und können je nach Substitutionsmuster von $R_3$ auch weitere Asymmetriezentren besitzen und können daher in verschiedenen stereochemischen Formen existieren. Als Beispiele seien genannt:

(-)-(1R,5R,9R)-N-(2-Furylmethyl)-5,9-dimethyl-2′-hydroxy-6,7-benzomorphan (a-Form),
(+)-(1S,5S,9S)-N-(2-Furylmethyl)-5,9-dimethyl-2′-hydroxy-6,7-benzomorphan (a-Form),
(-)-(1R,5R,9S)-N-(2-Furylmethyl)-5,9-dimethyl-2′-hydroxy-6,7-benzomorphan (β-Form),
(+)-(1S,5S,9R)-N-(2-Furylmethyl)-5,9-dimethyl-2′-hydroxy-6,7-benzomorphan (β-Form).

Gegenstand der Erfindung ist die Verwendung von Benzomorphanen der allgemeinen Formel I, in Form der einzelnen Stereoisomeren oder deren Mischungen sowie der entsprechenden physiologisch unbedenklichen Säureadditionssalze zur Zytoprotektion.

Es ist bekannt, daß nach systemischer Applikation von Glutamat Neuronen im Gehirn von Mäusen zerstört werden [S.M. Rothman und T.W. Olney, Trends in Neurosciences 10 (1987) 299]. Dieser Befund läßt den Schluß zu, daß Glutamat eine Rolle bei neurodegenerativen Erkrankungen spielt [R. Schwarcz und B. Meldrum, The Lancet 11 (1985) 140].

Weiterhin sind Substanzen wie z.B. Quisqualinsäure, Kainsäure, Ibotensäure, Glutaminsäure und N-Methyl-D-aspartat (NMDA) als exogene bzw. endogene Neurotoxine bekannt. Hinsichtlich der Neurotoxizität wirken diese Substanzen bezüglich der einzelnen Zelltypen selektiv, so daß bei Tieren durch spezifische Gehirnläsionen Funktionsausfälle induziert werden können. Diese sind mit jenen vergleichbar, welche im Zusammenhang mit Epilepsie und anderen neurodegenerativen Erkrankungen - wie zum Beispiel Huntington's Disease und Morbus Alzheimer - auftreten.

Ferner haben in vivo und in vitro Versuche gezeigt, daß die im Gehirn infolge von Hypoglykämie, Hypoxie, Anoxie und Ischämie auftretenden Zellschäden und Funktionsausfälle z.T. auf einer erhöhten synaptischen Aktivität beruhen, wobei die glutamaterge Synapse von besonderer Bedeutung ist. Substanzen und Ionen, welche die Aktivität des Glutamat-Rezeptors und des mit diesem Rezeptor verbundenen Ionenkanals hemmen - wie z.B. kompetitive und nicht-kompetitive Antagonisten excitatorischer Aminosäuren sowie Magnesium-Ionen ($Mg^{2+}$) - schützen Gehirnzellen vor hypoxischen bzw. ischämischen Schäden. Diese Befunde zeigen, daß der Glutamat-Rezeptor eine wichtige Rolle bei der Vermittlung des ischämischen Schadens spielt.

Biochemische und elektrophysiologische Studien lehren, daß der Rezeptor-Ionenkanal gegenüber Schwankungen der Magnesiumkonzentration hochempfindlich ist. Bei einem Abfallen der Magnesiumkonzentration kommt es im Hippocampus zu spontanen epileptischen Nachentladungen, die durch Antagonisten excitatorischer Aminosäuren gehemmt werden können.

Überraschenderweise wurde nun gefunden, daß Benzomorphane der allgemeinen Formel I eine zytoprotektive Wirkung aufweisen.

Die Herstellung dieser Benzomorphanderivate ist aus der DE-PS 21 05 743 und der DE-OS 28 28 039 sowie aus der Literatur [H. Merz und K. Stockhaus, J. Med. Chem. 22 (1979) 1475] bekannt. Ebenso ist bereits bekannt, daß derartige Verbindungen eine analgetische Wirksamkeit besitzen und als suchtfreie Analgetika sowie als Antitussiva therapeutisch angewandt werden können (DE-PS 21 05 743).

Als Testsystem für den Nachweis der zytoprotektiven Eigenschaften der Benzomorphanderivate dient der Hippocampusschnitt. Über Mikroelektroden werden die Schäfferkollaterale des sich in einer Perfusionskammer befindenden Hippocampusschnittes stimuliert und die auftretenden Summenpotentiale extrazellulär an den Pyramidenzellen der CAI-Region abgeleitet [H.L. Haas, B. Schaerer und M. Vosmansky, J. Neuroscience Meth. 1 (1979) 323].

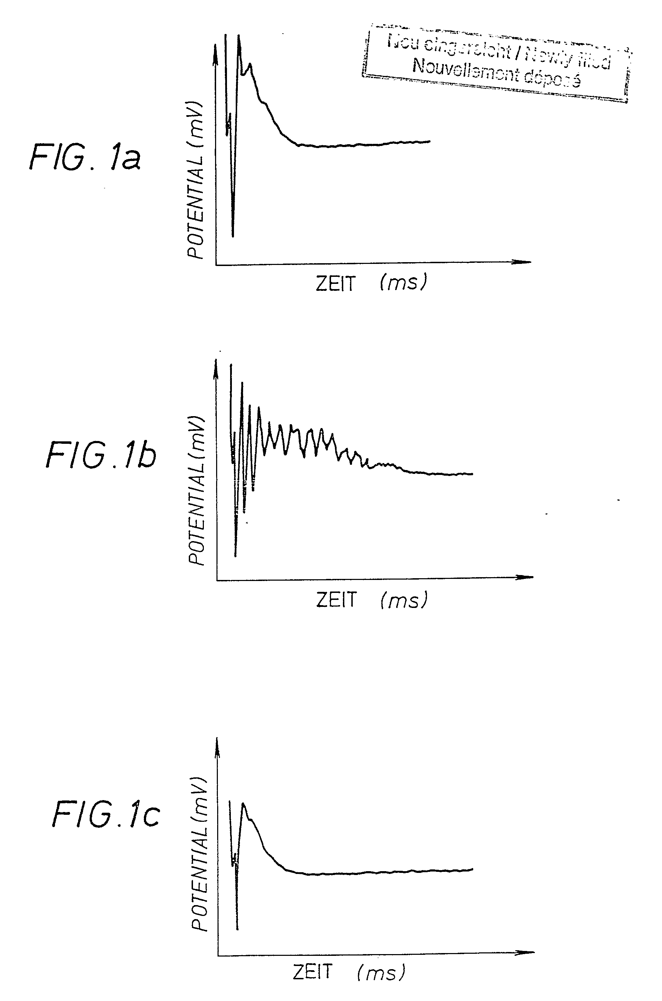

Eine typische Stimulation in einem magnesiumhaltigem Medium ist in Abb. 1a dargestellt. - Abb. 1b zeigt multiple epileptische Entladungen in einem magnesiumfreien Medium. Diese Entladungen werden - wie Abb. 1c belegt - beispielsweise durch Zugabe von 100 mmol (-)-(1R,5R,9R)-N-(2-Furylmethyl)-5,9-dimethyl-2′- hydroxy-6,7-benzomorphan gehemmt.

Die zytoprotektive Wirkung der Benzomorphanderivate, die unter die allgemeine Formel I fallen, wurde außerdem auch in Bezug auf Proteinsynthese und Neurotransmitter-Freisetzung im Hippocampusschnitt nachgewiesen.

Rezeptorbindungstests zeigen weiterhin, daß die offenbarten Benzomorphanderivate nicht-kompetitive Glutamat-Rezptorantagonisten sind.

Weiterhin wurde die zytoprotektive Wirkung der Benzomorphanderivate der allgemeinen Formel I an der Maus - in vivo - durch Hemmung der N-Methyl-D-asparaginsäure induzierten Letalität nachgewiesen [J.D. Leander et al., Brain Research 448 (1988) 115].

Diese Ergebnisse liefern einen Beleg dafür, daß die Benzomorphanderivate der allgemeinen Formel I bei neurodegenerativen Erkrankungen sowie Gehirnischämie verschiedener Genese eingesetzt werden können. Hierunter fallen beispielsweise:

Status Epileptikus, Hypoglykämie, Hypoxie, Anoxie, Gehirntrauma, Gehirnoedem, amyotrope laterale Sklerose, Huntington's Disease, Morbus Alzheimer, Hypotonie, Herzinfarkt, Gehirn-Schlaganfall und perinatale Asphyxie.

Die Benzomorphanderivate der allgemeinen Formel I sowie ihre pharmakologisch unbedenklichen Säureadditionssalze können in bekannter Weise in die üblichen Formulierungen - wie Tabletten, Dragees, Pillen, Granulate, Aerosole, Siruppe, Emulsionen, Suspensionen und Lösungen unter Verwendung inerter pharmazeutisch geeigneter Trägerstoffe oder Lösungsmittel überführt werden. Hierbei soll der Anteil der pharmazeutisch wirksamen Verbindung(en) jeweils im Bereich von 0,5 bis 90 Gew.-% der Gesamtzusammensetzung liegen, d.h. in Mengen, die ausreichend sind, um den unten angegebenen Dosierungsbereich zu erreichen.

Die Formulierungen werden zum Beispiel durch Verstrecken der Wirkstoffe mit Lösungsmitteln und/oder Trägerstoffen, gegebenenfalls unter Verwendung von Emulgiermitteln und/oder Dispergiermitteln hergestellt, wobei beispielsweise bei der Verwendung von Wasser als Verdünnungsmittel gegebenenfalls organische Lösungsmittel als Lösungsvermittler bzw. Hilfslösungsmittel eingesetzt werden können.

Als Hilfsstoffe seien beispielsweise Wasser, pharmazeutisch unbedenkliche organische Lösungsmittel, wie Paraffine (z.B. Erdölfraktionen), öle pflanzlichen Ursprungs (z.B. Erdnuß- oder Sesamöl), mono- oder polyfunktionelle Alkohole (z.B. Ethanol oder Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure und Silikate), Zucker (z.B. Rohr-, Milch- und Traubenzucker), Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise oral oder parental insbesondere perlingual oder intravenös. Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Die Dosierung für die orale Anwendung liegt bei 1-300 mg vorzugsweise zwischen 5 und 150 mg.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Ferner können die Verbindungen der allgemeinen Formel I bzw. deren Säureadditionssalze auch mit andersartigen Wirkstoffen kombiniert werden.

## Formulierungsbeispiele

### Tabletten

1. Die Tablette enthält folgende Bestandteile:

| | |
|---|---|
| Wirkstoff gemäß Formel I | 0,020 Teile |
| Stearinsäure | 0,010 " |
| Dextrose | 1,890 " |
| gesamt | 1,920 Teile |

### Herstellung

Die Stoffe werden in bekannter Weise zusammengemischt und die Mischung zu Tabletten verpreßt, von denen jede 1,92 g wiegt und 20 mg Wirkstoff enthält.

| Amupullenlösung | |
|---|---|
| Zusammensetzung | |
| Wirkstoff gemäß Formel I | 1,0 mg |
| Natriumchlorid | 45,0 mg |
| Aqua pro inj. | ad 5,0 ml |

### Herstellung:

Der Wirkstoff wird bei Eigen-pH oder gegebenenfalls bei pH 5,5 bis 6,5 in Wasser gelöst und mit

Natriumchlorid als Isotonanz versetzt. Die erhaltene Lösung wird pyrogenfrei filtriert und das Filtrat unter aseptischen Bedinungen Ampullen abgefüllt, die anschließend sterilisiert und zugeschmolzen werden. Die Ampullen enthalten 1 mg, 5 mg und 10 mg Wirkstoff.

Suppositorien

Jedes Zäpfchen enthält:

| | |
|---|---|
| Wirkstoff gemäß Formel I | 1,0 Teile |
| Kakaobutter (Schmp.: 36-37° C) | 1200,0 " |
| Carnaubawachs | 5,0 " |

Hestellung

Kakaobutter und Carnaubawachs werden zusammengeschmolzen. Bei 45° C gibt man den Wirkstoff hinzu und rührt bis eine komplette Dispersion entstanden ist. Die Mischung wird in Formen entsprechender Größe gegoßen und die Zäpfchen zweckmäßig verpackt.

## Ansprüche

1) Verwendung von Benzomorphanderivaten der allgemeinen Formel I

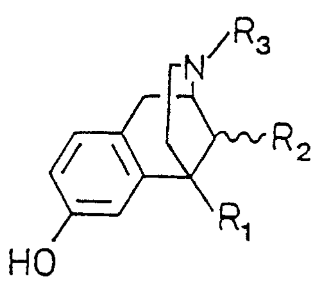

I

worin

$R_1$ Methyl, Ethyl;

$R_2$ Methyl, Ethyl;

$R_3$ 2-Methoxyethyl, 2-Methoxypropyl, 2-Furylmethyl (Furfuryl), 2-Tetrahydrofurylmethyl (Tetrahydrofurfuryl),

$$CH_2-\underset{R_5}{\overset{R_4}{C}}-O-R_6;$$

$R_4$ Wasserstoff, Methyl;

$R_5$ Wasserstoff, Methyl;

$R_6$ Methyl, Ethyl, Propyl, Isopropyl, Phenyl

bedeuten können;
deren α und β-Formen sowie deren enantiomere Formen und den entsprechenden pharmakologisch unbedenklichen Säureadditionssalzen zur Zytoprotektion.

2) Verwendung von Benzomorphanen gemäß Anspruch 1 zur Behandlung von Gehirnischämien verschiedener Genese, Epilepsie und neurodegenerativen Erkrankungen.

3) Verwendung von (-)-(1R,5R,9R)-N-(2-Furylmethyl)-5,9-dimethyl-2′-hydroxy-6,7-benzomorphan und seiner pharmakologisch unbedenklichen Säureadditionssalzen zur Zytoprotektion.

4) Verwendung von (-)-(1R,5R,9R)-N-(2-Furylmethyl)-5,9-dimethyl-2′-hydroxy-6,7-benzomorphan nach Anspruch 3 zur Behandlung von Gehirnischämien verschiedener Genese, Epilepsie und neurodegenerativen Erkrankungen.

Neu eingereicht / Newly filed
Nouvellement déposé

FIG. 1a

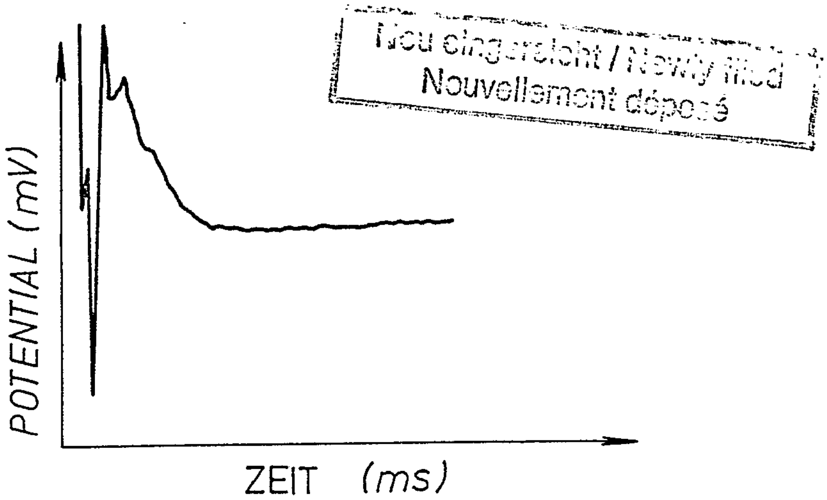


FIG. 1b

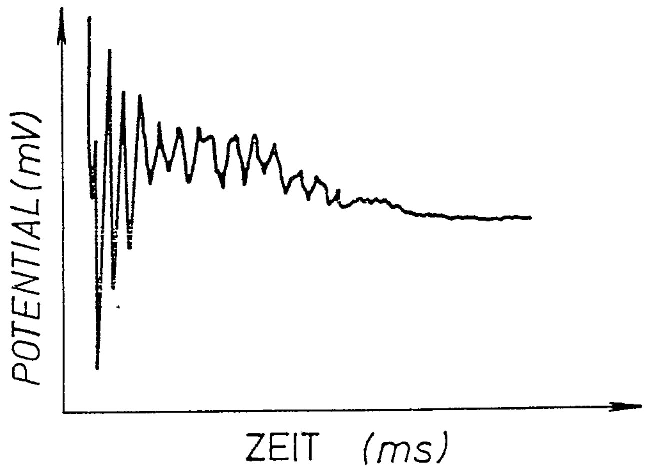


FIG. 1c